# EUROPEAN PATENT SPECIFICATION

(11) **EP 3 521 278 B1**
(45) Date of publication and mention of the grant of the patent: **15.04.2020**
(21) Application number: 19160019.6
(22) Date of filing: 25.01.2017
(51) Int. Cl.: C07D 251/60

(54) **METHOD FOR REVAMPING A HIGH PRESSURE MELAMINE PLANT**
VERFAHREN ZUR UMGESTALTUNG EINER HOCHDRUCKMELAMINANLAGE
PROCÉDÉ DE RESTRUCTURATION D'UNE INSTALLATION DE MÉLAMINE SOUS HAUTE PRESSION

(30) Priority: 19.02.2016 EP 16156505
(43) Date of publication of application: 07.08.2019
(62) Divisional of application: 17701154.1
(73) Proprietor: CASALE SA, 6900 Lugano (CH)
(72) Inventor: DI CARLO, Gabriele, 6900 Lugano (CH); SCOTTO, Andrea, 6932 Breganzona (CH); GAMBA, Simone, 24040 Pagazzano (BG) (IT)
(74) Representative: Zardi, Marco

(56) References cited:
- EP-A1- 1 054 006
- WO-A2-2009/080176

## Description

### Field of application

The invention relates to the field of melamine production from urea and relates in particular to a method of revamping of the purification section of a high pressure melamine plant.

### Prior Art

The processes for the synthesis of melamine from urea comprise low pressure catalytic processes and high pressure non-catalytic processes, typically above 7 MPa. These processes are well-known in the art.

A conventional type of plant for the synthesis of melamine using the high pressure non-catalytic process comprises a high pressure section substantially limited to a reactor, called melamine reactor, wherein the urea melt feed is converted into raw melamine with addition of heat.

Conversion of urea into melamine also generates gases mainly consisting of ammonia and carbon dioxide (so-called "off-gas") and a number of by-products (mainly OATs and polycondensates). The reaction products (melamine and off-gas), the by-products and the unreacted urea are further processed in a purification section operating at significantly lower pressure and temperature and generally comprising a quencher, a stripper and an absorber.

In the quencher, raw melamine is dissolved in an ammonia aqueous solution to separate off-gas from melamine. The off-gas are conveyed to a condensation section and melamine is supplied to the stripper, preferably a steam stripper, for removal of residual off-gas. The latter are absorbed in water inside the absorber and melamine is subjected to further purification in downstream equipment.

This configuration is widely used, but has a number of drawbacks.

Generally, the melamine plant is combined with a urea plant which produces the urea melt feed, starting from NH₃ and CO₂. The off-gas extracted from the quencher of the melamine plant are thus generally recycled to the combined urea plant. However, this implies condensation of the off-gas into a solution containing NH₃ and CO₂ suitable to be conveyed back to the urea plant, and a related condensation section.

Moreover, in order to avoid ammonia losses from the absorber and minimize the carbon dioxide content in the melamine solution leaving the stripper, a sequence of desorption and absorption occurring, respectively, in the stripper and the subsequent absorber is strongly required. However, it implies a high steam consumption inside the stripper, which is very disadvantageous in terms of costs.

WO 2009/080176 discloses a non-catalytic process for the preparation of melamine wherein a gaseous mixture of vaporized melamine and reaction off-gases is quenched by contact with an aqueous ammonium carbamate solution.

### Summary of the invention

The purpose of the invention is to avoid the above drawbacks of the prior art.

Said purpose is achieved with a method of revamping of a melamine purification section of a high pressure melamine plant according to the claims.

The term "high pressure melamine plant" denotes a plant for the synthesis of melamine using a high pressure non-catalytic process, which comprises a synthesis section operating at a high pressure, which is typically greater than 7 MPa, and a purification section operating at a lower pressure, which is typically 2 to 3 MPa.

The high pressure melamine plant comprises a synthesis section and a melamine purification section, wherein said synthesis section provides a first melamine-containing stream.

The present invention concerns the revamping of the purification section of a prior art melamine plant comprising:
a quencher fed with a first melamine-containing stream collected from the synthesis section of the plant and an aqueous solution comprising ammonia and carbon dioxide, wherein melamine is dissolved and off-gas are extracted;
a stripper fed with a second melamine-containing stream from said quencher and with a stripping medium, said stripping medium being preferably steam, wherein purified melamine is obtained and vapours are extracted, said vapours being added with a stream of water once extracted from the stripper;
a first heat exchanger, wherein the vapours added with said stream of water are at least partially condensed by heat exchange with an aqueous solution comprising ammonia and carbon dioxide, providing a condensed stream;
an absorber fed with said condensed stream and a carbonate solution, which provides an aqueous solution comprising ammonia and carbon dioxide, said aqueous solution being at least partially used for condensing the vapours in said first heat exchanger, further heated in a second heat-exchanger and recycled to the quencher. Said carbonate solution is preferably recycled from an ammonia separation section.

Said method of revamping is characterized by: installation of a line for at least partially feeding a stream of water (possibly added with ammonia) to the first heat exchanger for condensing said vapours; installation of a line for at least partially sending the stream of water leaving the first heat exchanger to the quencher; installation of a line for at least partially exporting the aqueous solution provided by said absorber from the purification section of the plant; discontinuing the line for feeding said aqueous solution to the first heat exchanger, for subsequent feeding to the second exchanger and recycle to the quencher; discontinuing the line for adding water to the vapours extracted from the stripper; discontinuing the line for introducing the carbonate solution into the absorber.

Preferably, said stream of water at least partially supplied to the first heat exchanger is a recycle stream coming from the plant. More preferably, a line is installed for injecting ammonia into said stream of water.

According to a preferred embodiment, a line bypassing the first heat exchanger is installed for supplying a portion of said stream of water, possibly added with ammonia, to the quencher. Even more preferably, said line is installed downstream of the line for injecting ammonia into said stream of water, the ammonia being able to maintain an adequate pH value in the quencher preventing the formation of OATs.

Advantageously, the line of water leaving the first heat exchanger passes through said second heat exchanger for further heating and then is supplied to the quencher.

According to an embodiment of the prior art, a further portion of the aqueous solution extracted from the absorber is directly supplied to the quencher and, according to the method of the invention, said further line is discontinued.

Advantageously, the line for extracting the off-gas from the quencher is discontinued due to the lack of carbon dioxide and ammonia leaving the quencher as gaseous stream.

Said second melamine-containing stream from the quencher is preferably subjected to further purification treatment downstream said purification section.

More preferably, a pump is installed downstream of the quencher for suitably pressurizing said second melamine-containing stream before being subjected to said further purification.

According to particularly advantageous embodiments of the invention, the above method of revamping concerns the purification section of a high pressure melamine plant of the prior art provided with a synthesis section comprising a melamine reactor, a stripping reactor and a scrubber, as disclosed in the patent application No. EP15154205.7.

The vapours extracted from the stripper may contain ammonia and carbon dioxide.

The term "first melamine-containing stream" refers to a raw melamine melt and the term "second melamine-containing stream" refers to a melamine solution.

Preferably, said first melamine-containing stream is free, or substantially free, of melamine offgas. The synthesis section of the high pressure melamine plant may comprise a melamine reactor, a stripping reactor and a scrubber.

The term of melamine offgas denotes the offgas released from the synthesis of melamine, predominantly made of carbon dioxide and ammonia. The reference to a stream which is free or substantially free of melamine offgas denotes that the stream contains no or a negligible amount of said offgas in the gaseous phase, since the offgas are removed in the synthesis section of the high pressure melamine plant.

The offgas-free melamine stream may contain a small amount of offgas dissolved in the liquid phase, particularly ammonia, since carbon dioxide is at least in part separated from liquid melamine in the stripping stage. A melamine-containing stream free from offgas is preferably generated by the above mentioned setup of a melamine reactor, stripping reactor and scrubber.

The very low amount or absence of offgas in the first melamine-containing stream, particularly the absence of carbon dioxide, reduces the duty and cost of the purification section. Accordingly, the purification section is aimed basically to quench and dilute the first melamine-containing stream and is not required to condense a large amount of gaseous ammonia and carbon dioxide.

The term "purified melamine" refers to the melamine melt obtained in the stripping reactor, which has higher purity than the raw melamine obtained in the melamine reactor, but which needs be further purified in the subsequent purification section.

The term "high pressure section" denotes that the melamine reactor, the stripping reactor and the scrubber operate substantially at the same nominal pressure, said pressure being greater than 7 MPa and preferably greater than 10 MPa, for example 11 MPa.

The present invention has the following advantages.

First, the undesired sequence of desorption and absorption taking place, respectively, in the stripper and the absorber is avoided because the ammonia content in the absorber is minimized, as well as the carbon dioxide content in the effluent from the stripper. As a consequence, the steam consumption inside the stripper is significantly reduced or eliminated in the case the revamping provides for the stripper elimination.

A further advantage is that the liquid stream leaving the quencher is not saturated with ammonia and carbon dioxide, which provides for less off-gas extracted from the subsequent stripping and a lower content of ammonia and carbon dioxide in the aqueous solution collected from the absorber. An even lower content of ammonia and carbon dioxide in the liquid stream leaving the quencher justifies the dismissing of the stripper and the absorber.

Moreover, the quencher does not provide any gaseous stream and no off-gas are recovered therefrom. As a consequence, an off-gas condensation unit is no longer required.

Another advantage is that a portion of the aqueous solution from the absorber is exported from the purification section for further use in the process, thanks to the fact that the water input for the quencher is provided by a stream of water recycled from the plant.

The advantages will emerge even more clearly with the aid of the detailed description below relating to preferred embodiments.

### Description of the figures

Fig. 1 is a block scheme of a melamine plant.
Fig. 2 is a schematic diagram of the high-pressure section of the plant according to Fig. 1.
Fig. 3 is a block scheme of a melamine plant according to the prior art.
Fig. 4 is a block scheme of a melamine plant after revamping of the plant of Fig. 3, according to the invention.

### Detailed description

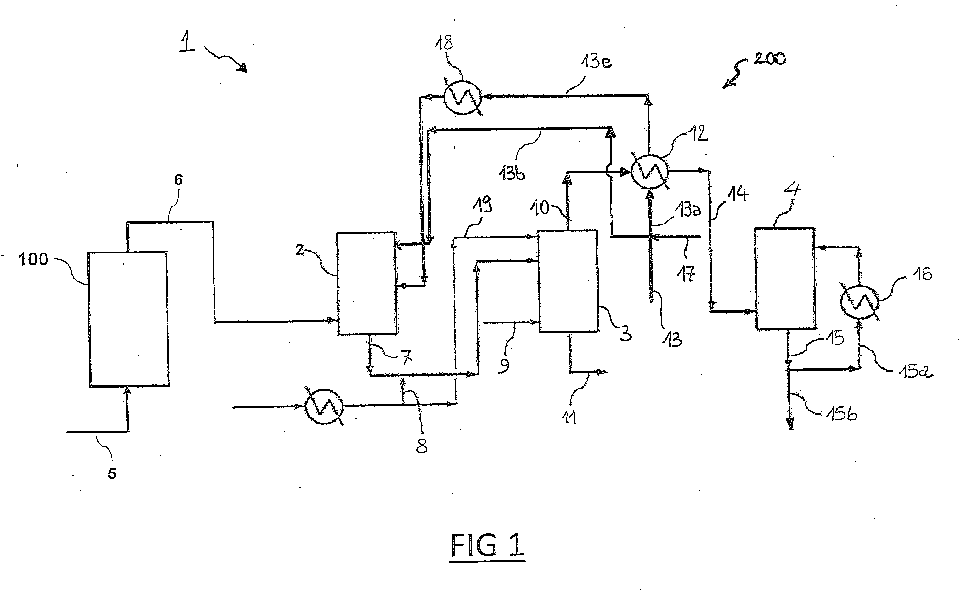

With reference to Fig. 1, the melamine plant 1 comprises a high-pressure section 100 and a purification section 200 which operates at significantly lower pressure and temperature.

The high-pressure section 100 is fed with urea melt 5 and produces melamine 6. As the melamine 6 leaves the high-pressure section 100, the pressure is lowered from above 7 MPa to 0.4-2 MPa and melamine is supplied to the purification section 200.

Said purification section 200 essentially comprises a quencher 2, a stripper 3 and an absorber 4. In the quencher 2, melamine 6 is treated at around 160 °C with aqueous solutions of ammonia 13c, 13b to dissolve melamine. Melamine is collected from the bottom of the quencher 2 as an aqueous solution 7, further diluted with a stream of water 8 coming from the plant and fed to the stripper 3.

The stripper 3 is further fed with steam 9, which may come from the plant, and with a further stream of water 19 coming from the plant. In the stripper 3, vapours 10 are extracted from the top and an ammonia- and carbon dioxide- free melamine solution 11 is collected from the bottom. Said solution 11 is then subjected to further purification, comprising for example: filtration, clarification with activated carbon, crystallization, solid separation of the melamine crystals and drying.

The vapours 10 are then sent to a heat exchanger 12, where they are condensed by thermal exchange with an aqueous solution of ammonia 13a.

The condensed vapours 14 are fed into the absorber 4, which provides an aqueous solution 15. Said aqueous solution is split into two portions: the first portion 15a is passed through a cooler 16 and recirculated inside the absorber 4 and the second portion 15b is exported from the purification section 200 and preferably recycled to the plant.

A stream of water 13 recycled from the plant and an ammonia stream 17 are mixed to provide an aqueous ammonia solution, which is split into portions 13a, 13b. The first portion 13a is fed to the above heat exchanger 12, where it is preheated to provide stream 13c, and the second portion 13b is supplied to the quencher 2.

Said stream 13c of preheated aqueous solution is fed to another heat exchanger 18, where it is further heated, and supplied to the quencher 2.

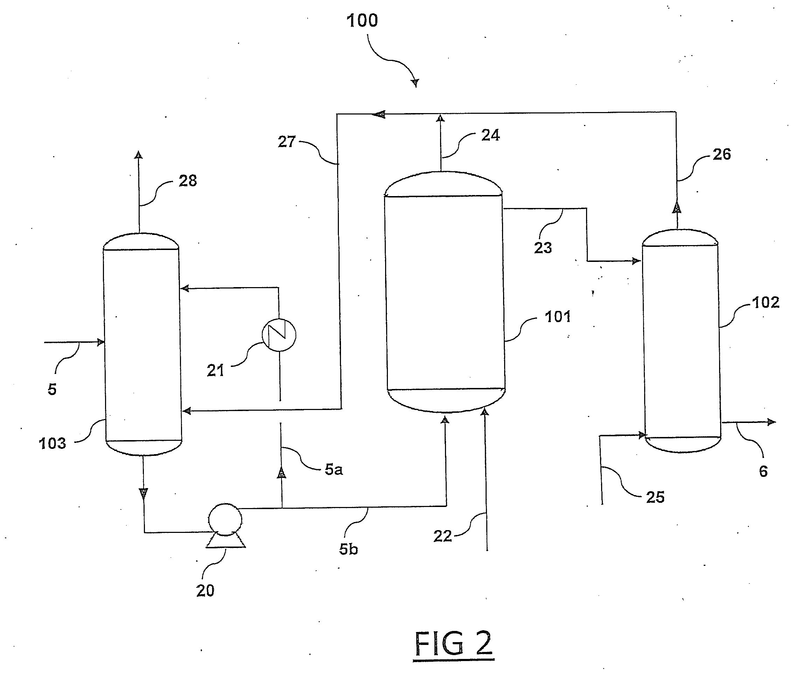

Fig. 2 shows the high-pressure section 100 of the plant 1 of Fig. 1, which comprises essentially a first melamine reactor 101, a second stripping reactor 102 and a scrubber 103.

In particular, the scrubber 103 is fed with the urea melt 5. The urea melt leaving the scrubber passes through a pump 20 and a first portion 5a of said urea melt is recirculated inside the scrubber 103 after passing through a heat exchanger 21, where it is advantageously cooled producing vapour, and a second portion 5b of said urea melt is supplied to the melamine reactor 101.

The melamine reactor 101 is also fed with a heat-carrier fluid 22 which supplies heat to the melamine reactor 101 in order to promote the endothermic reaction for conversion of urea into melamine. Typically said heat-carrier fluid 22 consists of molten salts which circulate inside suitable heating pipes of the reactor.

Raw melamine is separated as stream 23 and off-gas are extracted as stream 24.

Said raw melamine 23 is sent to the second stripping reactor 102, which is further fed with gaseous ammonia 25 as stripping agent. The purified melamine 6 leaving the stripping reactor 102 is fed to the quencher 2 of the purification section 200 of the plant, generally operating at a lower pressure than the synthesis section 100, and the off-gas 26 extracted therefrom are combined with the off-gas 24 from the melamine reactor 101, providing a stream 27.

Said stream 27 is introduced into the scrubber 103, where it undergoes washing with the urea melt streams 5 and 5a, respectively feeding and recirculating streams. A high pressure stream of anhydrous off-gas 28, mainly composed of ammonia and carbon dioxide, is extracted from the scrubber 103.

For example, the urea melt 5 is produced in a urea plant connected to the melamine plant comprising the section 100. The off-gas 28, in view of their content of NH3 and CO2 (which constitute the reagents for obtaining urea), are preferably recycled to said urea plant.

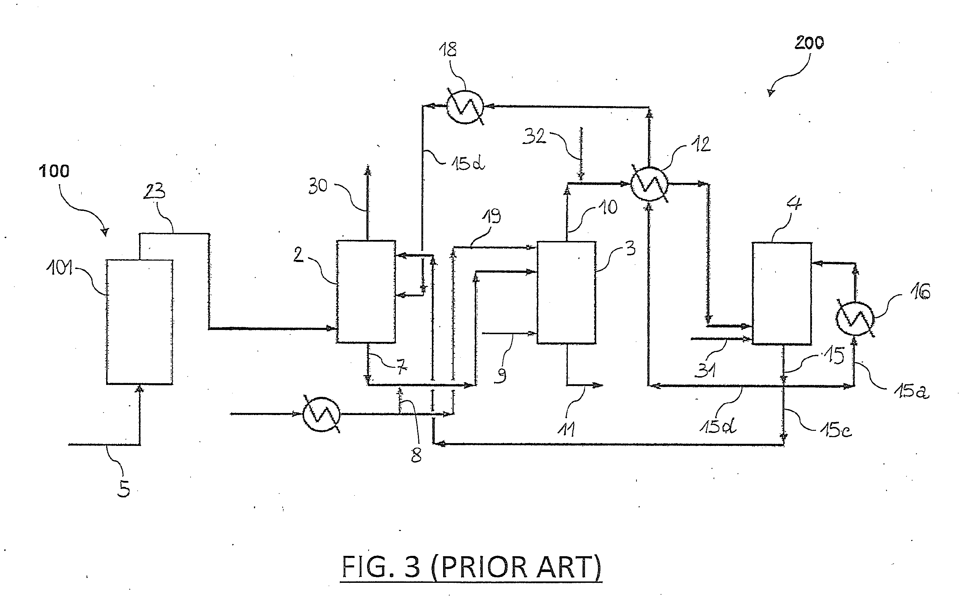

Fig. 3 shows a melamine plant according to the prior art, comprising a high-pressure section 100 and a purification section 200.

The high-pressure section 100 essentially comprises the melamine reactor 101, which is fed with the urea melt 5 and produces a stream 23 containing melamine and off-gas, which is supplied to the purification section 200.

The purification section 200 essentially comprises a quencher 2, a stripper 3 and an absorber 4. In the quencher 2, which is fed with said stream 23 containing melamine and off-gas and an aqueous solution 15d comprising ammonia and carbon dioxide, melamine 7 is collected from the bottom and off-gas 30 are extracted from the top.

The off-gas 30 leaving the top of the quencher 2 are saturated with water and conveyed to a condensation section (not shown); melamine 7 is diluted with a stream of water 8 and fed to the stripper 3, which is supplied with steam 9 and a further stream of water 19.

Vapours 10, which contain ammonia and carbon dioxide, are removed from the top of the stripper 3 and an ammonia- and carbon dioxide- free melamine solution 11 is collected from the bottom thereof. Said solution 11 is then subjected to further purification.

The vapours 10 are added with an amount of water 32, preferably recycled from the plant, condensed in a heat exchanger 12 and then absorbed in water within the absorber 4.

Said absorber is also fed with a carbonate solution 31, providing an aqueous solution 15. Said aqueous solution 15 is split into three portions: the first portion 15a is passed through a cooler 16 and recirculated inside the absorber 4, the second portion is recycled to the quencher 2 through a first line 15c and the third portion is recycled to the quencher 2 through a second line 15d.

The first line 15c provides direct supply of the above solution to the quencher 2, while the second line 15d provides: passage of the solution through the above mentioned heat exchanger 12 for condensing the off-gas 10 extracted from the stripper 3; passage through a further heat exchanger 18 where it is further heated; supply to the quencher 2.

Said plant of the prior art is advantageously revamped to provide the melamine plant illustrated in Fig. 1, by means of the following operations:
installation of a line 13 for recycling a stream of water from the plant;
installation of a line 17 for injecting ammonia into said stream of water;
installation of a line 13a for feeding a first portion of said stream of water containing ammonia to the first heat exchanger 12, wherein said stream of water 13a is preheated;
installation of a line 13b for feeding a second portion of said stream of water containing ammonia to the quencher 2;
installation of a line 13c for sending the preheated water to the second heat exchanger 18 and then to the quencher 2;
installation of a line 15b for at least partially exporting the aqueous solution provided by said absorber 4 from the purification section 200 of the plant;
discontinuing the line 15d for feeding said aqueous solution to the first heat exchanger 12, for subsequent feeding to the second-exchanger 18 and recycle to the quencher 2;
discontinuing the line 15c for directly supplying said aqueous solution to the quencher 2;
discontinuing the line 32 for adding said stream of water 32 to the vapours 10 extracted from the stripper 3;
discontinuing the line 31 for introducing the carbonate solution into the absorber 4;
discontinuing the line 30 for extracting the off-gas 30 from the quencher 2.

According to this embodiment, the revamping of the purification section 200 is carried out after revamping of the high pressure synthesis section to provide the synthesis section of Fig. 2.

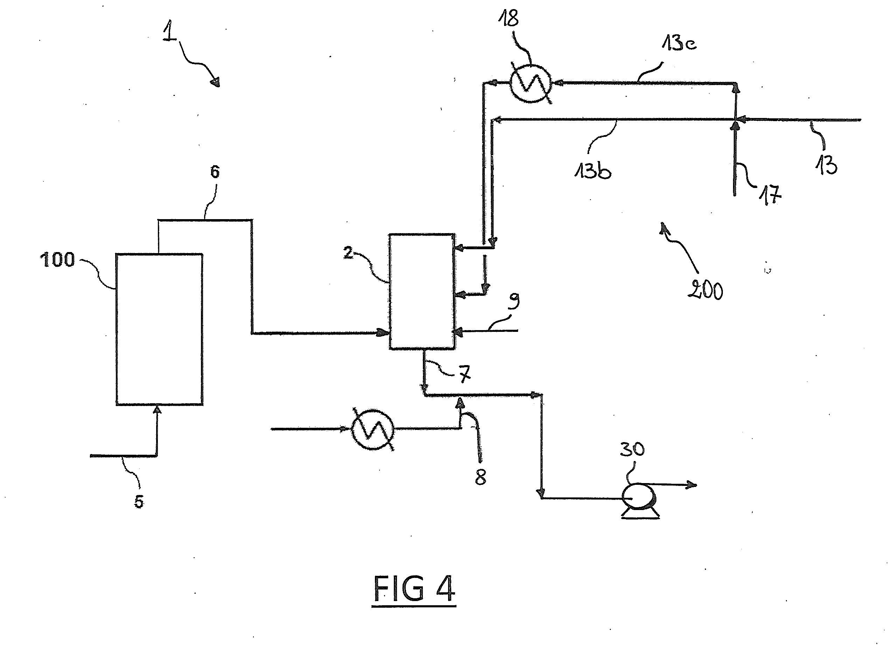

According to another embodiment, which is not part of the present invention, the plant of the prior art is revamped to provide the melamine plant illustrated in Fig. 4, by means of the following operations:
redirecting the stripping medium (9) to the quencher (2);
installation of a line 13 for recycling a stream of water from the plant;
installation of a line 17 for injecting ammonia into said stream of water;
installation of a line 13c for feeding a first portion of said stream of water containing ammonia to the second heat exchanger 18 and then to the quencher 2;
installation of a line 13b for feeding a second portion of said stream of water containing ammonia to the quencher 2;
installation of a line for exporting said second melamine-containing stream 7 from said quencher 2, said stream 7 being sent to a further purification section (not shown);
installation of a pump 30 downstream the quencher 2, which receives said second melamine-containing stream 7 before being sent to said further purification section;
dismissing the stripper 3, the first heat exchanger 12, the absorber 4 and the cooler 16;
discontinuing the inlet and outlet lines to / from the stripper 3, the first heat exchanger 12, the absorber 4 and the cooler 16;
discontinuing the line 30 for extracting the off-gas 30 from the quencher 2.

According to this embodiment, the revamping of the purification section 200 is carried out after revamping of the high pressure synthesis section to provide the synthesis section of Fig. 2.

## Claims

1. A method for revamping a melamine purification section (200) of a high pressure melamine plant comprising:
a quencher (2) fed with a first melamine-containing stream (23), which is a raw melamine melt, from the synthesis section (100) and an aqueous solution (15d) comprising ammonia and carbon dioxide, wherein melamine (7) is dissolved and off-gas (30) are extracted;
a stripper (3) fed with a second melamine-containing stream (7) from said quencher (2) and with a stripping medium (9), wherein purified melamine (11) is obtained and vapours (10) are extracted, said vapours being added with a stream of water (32) once extracted from the stripper (3);
a first heat exchanger (12), wherein the vapours (10) added with said stream of water (32) are at least partially condensed by heat exchange with an aqueous solution (15d) comprising ammonia and carbon dioxide, providing a condensed stream (14);
an absorber (4) fed with said condensed stream (14) and a carbonate solution (31) and providing an aqueous solution (15) comprising ammonia and carbon dioxide, which is at least partially used for condensing the vapours (10) in said first heat exchanger (12), heated in a second heat-exchanger (18) and recycled to the quencher (2),
said method being **characterized by**
installation of a line (13a) for at least partially feeding a stream of water, possibly added with ammonia, to the first heat exchanger (12) for condensing said vapours (10);
installation of a line (13c) for at least partially sending the water leaving said heat exchanger (12) to the quencher (2);
installation of a line (15b) for at least partially exporting the aqueous solution provided by said absorber (4) from the purification section (200) of the plant;
discontinuing the line (15d) for feeding said aqueous solution to the first heat exchanger (12), for subsequent feeding to the second-exchanger (18) and recycle to the quencher (2);
discontinuing the line (32) for adding said stream of water (32) to the vapours (10) extracted from the stripper (3);
discontinuing the line (31) for introducing the carbonate solution into the absorber (4).

2. A method according to claim 1, wherein a line (13b) is installed for feeding a portion of said stream of water, possibly added with ammonia, to the quencher (2) bypassing the first heat exchanger (12).

3. A method according to claim 1 or 2, a portion (15c) of the aqueous solution extracted from the absorber (4) being directly supplied to the quencher (2), the method being **characterized by** discontinuing the line for directly supplying said aqueous solution to the quencher.

4. A method according to any one of claims 1 to 3, wherein the line (30) for extracting the off-gas (30) from the quencher (2) is discontinued.

5. A method according to any of claims 1 to 4, wherein a line (17) for injecting ammonia to said stream of water (13) is installed.

## Patentansprüche

1. Verfahren zum Umgestalten eines Melaminreinigungsabschnitts (200) einer Hochdruck-Melaminanlage, umfassend:
einen Quencher (2), dem ein erster melaminhaltiger Strom (23), der eine rohe Melaminschmelze ist, von dem Syntheseabschnitt (100) und eine wässrige Lösung (15d), die Ammoniak und Kohlendioxid umfasst, zugeführt wird, worin Melamin (7) gelöst und Abgas (30) extrahiert wird;
einen Stripper (3), dem ein zweiter melaminhaltiger Strom (7) von dem Quencher (2) und ein Strippmedium (9) zugeführt wird, worin gereinigtes Melamin (11) erhalten wird und Dämpfe (10) extrahiert werden, wobei den Dämpfen nach der Extraktion aus dem Stripper (3) ein Strom von Wasser (32) zugegeben wird;
einen ersten Wärmetauscher (12), worin die Dämpfe (10) mit dem zugegebenen Strom von Wasser (32) durch Wärmetausch mit einer wässrigen Lösung (15d), die Ammoniak und Kohlendioxid umfasst, wenigstens teilweise kondensiert werden, um einen kondensierten Strom (14) bereitzustellen;
einen Absorber (4), dem der kondensierte Strom (14) und eine Carbonatlösung (31) zugeführt wird und der eine wässrige Lösung (15), die Ammoniak und Kohlendioxid umfasst, bereitstellt, die wenigstens zum Teil zum Kondensieren der Dämpfe (10) in dem ersten Wärmetauscher (12) verwendet, in einem zweiten Wärmetauscher (18) erhitzt und zu dem Quencher (2) rückgeführt wird,
wobei das Verfahren **gekennzeichnet ist durch**
Einbauen einer Leitung (13a) zum wenigstens teilweisen Zuführen eines Stroms von Wasser, gegebenenfalls mit zugegebenem Ammoniak, zu dem ersten Wärmetauscher (12) zum Kondensieren der Dämpfe (10);
Einbauen einer Leitung (13c) zum wenigstens teilweisen Zuführen des Wassers, das aus dem Wärmetauscher (12) austritt, zu dem Quencher (2);
Einbauen einer Leitung (15b) zum wenigstens teilweisen Abführen der von dem Absorber (4) bereitgestellten wässrigen Lösung aus dem Reinigungsabschnitt (200) der Anlage;
Stilllegen der Leitung (15d) zum Zuführen der wässrigen Lösung zu dem ersten Wärmetauscher (12) zum nachfolgenden Zuführen zu dem zweiten Wärmetauscher (18) und Rückführen zu dem Quencher (2);
Stilllegen der Leitung (32) zum Zugeben des Stroms von Wasser (32) zu den aus dem Stripper (3) extrahierten Dämpfen (10);
Stilllegen der Leitung (31) zum Einführen der Carbonatlösung in den Absorber (4).

2. Verfahren gemäß Anspruch 1, wobei eine Leitung (13b) zum Zuführen eines Teils des Stroms von Wasser, gegebenenfalls mit zugegebenem Ammoniak, zu dem Quencher (2) unter Umgehung des ersten Wärmetauschers (12) eingebaut wird.

3. Verfahren gemäß Anspruch 1 oder 2, wobei ein Teil (15c) der aus dem Absorber (4) extrahierten wässrigen Lösung direkt dem Quencher (2) zugeführt wird, wobei das Verfahren durch Stilllegen der Leitung zum direkten Zuführen der wässrigen Lösung zu dem Quencher gekennzeichnet ist.

4. Verfahren gemäß einem der Ansprüche 1 bis 3, wobei die Leitung (30) zum Extrahieren des Abgases (30) aus dem Quencher (2) stillgelegt wird.

5. Verfahren gemäß einem der Ansprüche 1 bis 4, wobei eine Leitung (17) zum Einspritzen von Ammoniak in den Strom von Wasser (13) eingebaut wird.

## Revendications

1. Procédé pour moderniser une section de purification de mélamine (200) d'une usine de mélamine haute pression comprenant :
un module de désactivation (2) alimenté par un premier courant contenant de la mélamine (23), qui est une masse fondue de mélamine brute, provenant de la section de synthèse (100) et une solution aqueuse (15d) comprenant de l'ammoniac et du dioxyde de carbone, où la mélamine (7) est dissoute et des dégagements gazeux (30) sont extraits ;
un module de rectification (3) alimenté par un deuxième courant contenant de la mélamine (7) provenant dudit module de désactivation (2) et avec un milieu de rectification (9), où de la mélamine purifiée (11) est obtenue et des vapeurs (10) sont extraites, lesdites vapeurs étant additionnées d'un courant d'eau (32) une fois extraite du module de rectification (3) ;
un premier module d'échange de chaleur (12), où les vapeurs (10) additionnées dudit courant d'eau (32) sont au moins partiellement condensées par échange de chaleur avec une solution aqueuse (15d) comprenant de l'ammoniac et du dioxyde de carbone, ce qui donne un courant condensé (14) ;
un module d'absorption (4) alimenté par ledit courant condensé (14) et une solution de carbonate (31) et donnant une solution aqueuse (15) comprenant de l'ammoniac et du dioxyde de carbone, qui est au moins partiellement utilisée pour condenser les vapeurs (10) dans ledit premier module d'échange de chaleur (12), chauffée dans un deuxième module d'échange de chaleur (18) et recyclée vers le module de désactivation (2),
ledit procédé étant **caractérisé par**
l'installation d'une ligne (13a) pour introduire au moins en partie un courant d'eau, éventuellement additionné d'ammoniac, dans le premier module d'échange de chaleur (12) pour condenser lesdites vapeurs (10) ;
l'installation d'une ligne (13c) pour envoyer au moins en partie l'eau quittant ledit module d'échange de chaleur (12) vers le module de désactivation (2) ;
l'installation d'une ligne (15b) pour exporter au moins en partie la solution aqueuse fournie par ledit module d'absorption (4) depuis la section de purification (200) de l'usine ;
l'interruption de la ligne (15d) pour introduire ladite solution aqueuse dans le premier module d'échange de chaleur (12), pour une introduction subséquente dans le deuxième module d'échange (18) et un recyclage vers le module de désactivation (2) ;
l'interruption de la ligne (32) pour ajouter ledit courant d'eau (32) aux vapeurs (10) extraites du module de rectification (3) ;
l'interruption de la ligne (31) pour introduire la solution de carbonate dans le module d'absorption (4).

2. Procédé selon la revendication 1, dans lequel une ligne (13b) est installée pour introduire une partie dudit courant d'eau, éventuellement additionné d'ammoniac, dans le module de désactivation (2) contournant le premier échangeur de chaleur (12).

3. Procédé selon la revendication 1 ou 2, dans lequel une partie (15c) de la solution aqueuse extraite du module d'absorption (4) est directement fournie au module de désactivation (2), le procédé étant **caractérisé par** l'interruption de la ligne pour une fourniture directe de ladite solution aqueuse au module de désactivation.

4. Procédé selon l'une quelconque des revendications 1 à 3, dans lequel la ligne (30) pour extraire le dégagement gazeux (30) hors du module de désactivation (2) est interrompue.

5. Procédé selon l'une quelconque des revendications 1 à 4, dans lequel une ligne (17) pour injecter de l'ammoniac dans ledit courant d'eau (13) est installée.
